# EUROPÄISCHE PATENTANMELDUNG

(11) **EP 4 736 751 A1**
(43) Veröffentlichungstag der Anmeldung: **06.05.2026**
(21) Anmeldenummer: 24210852.0
(22) Anmeldetag: 05.11.2024
(51) Int. Cl.: A61B 5/02, A61B 5/026, A61B 5/0295, A61B 5/1455, A61B 5/00, G16H 50/30, A61B 5/024, A61B 5/022

(54) **COMPUTERIMPLEMENTIERTES VERFAHREN, COMPUTERPROGRAMMPRODUKT, COMPUTERLESBARES MEDIUM, SYSTEM, UND VERFAHREN ZUR BESTIMMUNG EINER KARDIOVASKULÄREN LEISTUNG**

(71) Anmelder: Marinow, Nikolai, 71083 Herrenberg (DE)
(72) Erfinder: Marinow, Nikolai, 71083 Herrenberg (DE)
(74) Vertreter: Hepp Wenger Ryffel AG

(57) **Zusammenfassung**

Die Erfindung betrifft ein computerimplementiertes Verfahren zur Bestimmung einer kardiovaskulären Leistung (2). Das Verfahren umfasst das Empfangen von einer Vielzahl von Messdaten (5) durch eine Kommunikationsschnittstelle (6) von einem Sensor (7) an einem Messabschnitt (41) der distalen Peripherie (4) des Patienten. Die Messdaten (5) geben Aufschluss über eine Perfusion des Blutflusses am Messabschnitt (41). Die Vielzahl der Messdaten (5) umfasst zumindest zwei verschiedene Messdaten (5), die zu unterschiedlichen Zeiten nach dem Wiederherstellen des Blutflusses zu der distalen Peripherie (4) detektiert wurden. Das Verfahren umfasst zudem die Bestimmung der kardiovaskulären Leistung (2), insbesondere des Herzzeitvolumens, anhand der Vielzahl von Messdaten und die Bereitstellung von Ausgangsdaten durch eine Ausgangsschnittstelle (8) umfassend die kardiovaskuläre Leistung (2).

## Beschreibung

Die Erfindung betrifft ein computerimplementiertes Verfahren, ein Computerprogrammprodukt, ein computerlesbares Medium, ein System, und ein Verfahren zur Bestimmung einer kardiovaskulären Leistung gemäss den unabhängigen Ansprüchen. Zudem betrifft die Erfindung ein computerimplementiertes Verfahren zum Trainieren eines maschinellen Lernmodells zur Bestimmung einer kardiovaskulären Leistung und einen Trainingsdatensatz gemäss den unabhängigen Ansprüchen.

Die kardiovaskuläre Leistung, insbesondere das Schlagvolumen und/oder das Herzzeitvolumen, ist ein wichtiges Mittel zur Beurteilung der Herzfunktion, der hämodynamischen Organperfusion, und ein Indikator für die Gesundheit des Herz-Kreislauf-Systems. Zudem kann durch eine Überwachung der kardiovaskulären Leistung die Indikation überprüft werden, wie der Einsatz von Medikamenten oder Therapiemassnahmen, etwa zur Behandlung einer Herzinsuffizienz, von Schock, oder von pulmonaler Hypertonie. Durch die Überwachung der kardiovaskulären Leistung kann etwa die postoperative Versorgung überwacht werden und/oder die bestimmungsgemässe Funktion von Implantaten, wie Herzschrittmachern kontrolliert werden.

US 2012/065514 A offenbart eine Vorrichtung, die eine pneumatische beaufschlagbare Manschette, und eine Doppler-Ultraschallsonde, die am Handgelenk anbringbar ist, zur Messung des Bluts umfasst. Diese Vorrichtung ermöglicht die Dopplergeschwindigkeit von Blut während einer Deflation der Manschette zu messen.

Eine Doppler-Ultraschallsonde ist jedoch kostenintensiv und schwierig exakt und zuverlässig zu positionieren, insbesondere bei körperlicher Belastung eines Patienten. Zudem offenbart oder lehrt US 2012/065514 keine Möglichkeit zur Bestimmung der kardiovaskulären Leistung.

Eine zuverlässige Bestimmung der kardiovaskulären Leistung ist zudem im Stand der Technik oftmals teuer, umständlich, komplex und/oder zeitaufwendig.

Es ist daher die Aufgabe der vorliegenden Erfindung diese und andere Nachteile des Stands der Technik zu überwinden und computerimplementierte Verfahren, ein Computerprogrammprodukt, ein computerlesbares Medium, ein System, ein Verfahren, und einen Trainingsdatensatz zur Verfügung zu stellen, welche eine einfache, kostengünstige, und zuverlässige Lösung der obengenannten Probleme ermöglichen. Zudem sollte die Bestimmung der kardiovaskulären Leistung eine breite Verfügbarkeit und Kompatibilität zu bestehenden Messverfahren/Messsystemen haben und vorzugsweise eine einfache und schnelle Bestimmung der kardiovaskulären Leistung bei körperlicher Belastung ermöglichen. Es ist somit möglich die kardiovaskuläre Leistung und damit einhergehende Diagnose(n) selbst bei starker Beanspruchung des Herzens zu bestimmen.

Das computerimplementierte Verfahren zur Bestimmung einer kardiovaskulären Leistung umfasst optional das Senden eines ersten Beaufschlagungsbefehls, vorzugsweise durch eine Kontrolleinheit, zur Beaufschlagung einer Manschette über einen systolischen Blutdruck des Patienten, um einen Blutfluss zu einer distalen Peripherie des Patienten zu unterbinden. Das computerimplementierte Verfahren umfasst optional das Senden eines zweiten Beaufschlagungsbefehls, vorzugsweise durch die Kontrolleinheit, zur Beaufschlagung der Manschette zu einem subsystolischen Blutdruck, um einen Blutfluss zu der distalen Peripherie zu ermöglichen. Das computerimplementierte Verfahren beinhaltet das Empfangen von einer Vielzahl von Messdaten durch eine Kommunikationsschnittstelle von einem Sensor an einem Messabschnitt der distalen Peripherie des Patienten. Die Vielzahl von Messdaten geben Aufschluss über eine Perfusion des Blutflusses am Messabschnitt. Die Vielzahl von Messdaten umfassen zumindest zwei verschiedene Messdaten, die zu unterschiedlichen Zeiten nach dem Wiederherstellen des Blutflusses zu der distalen Peripherie detektiert wurden. Das computerimplementierte Verfahren umfasst zudem die Bestimmung der kardiovaskulären Leistung anhand der Vielzahl von Messdaten und das Bereitstellen von Ausgangsdaten durch eine Ausgangsschnittstelle umfassend die kardiovaskuläre Leistung.

Die kardiovaskuläre Leistung kann das Herzzeitvolumen oder das Herzschlagvolumen sein.

Dieses computerimplementierte Verfahren ermöglicht es durch die Wiederherstellung der Perfusion des Blutflusses die kardiovaskuläre Leistung zu bestimmen, da diese sich erst nach einer geraumen Zeit nach Wiederherstellung des Blutflusses in einem Sättigungsbereich stabilisiert.

Eine optionale Steuerung der Manschette zur Druckbeaufschlagung ermöglicht zudem eine bessere Abstimmung des Verfahrens und eine einfachere Nutzeranwendung.

Der systolische Blutdruck eines Patienten liegt typischerweise etwa bei 120 mmHg, kann aber je nach Patienten auch etwa in einem Bereich von 90 - 180 mmHg liegen. Ein erster Beaufschlagungsbefehl zur Beaufschlagung einer Manschette über einen systolischen Blutdruck, oder auch arterieller Verschlussdruck, liegt somit oberhalb dieser Werte, damit die Manschette den Blutfluss in den Arterien zuverlässig unterbinden kann.

Der zweite Beaufschlagungsbefehl zur Beaufschlagung der Manschette zu einem subsystolischen Blutdruck, um einen Blutfluss zu der distalen Peripherie zu ermöglichen liegt somit unter den obengenannten Werten. Der zweite Beaufschlagungsbefehl kann den Blutfluss komplett freigeben, also lediglich eine geringe Druckbeaufschlagung oder einen Beaufschlagungsdruck von 0 mmHg oder zumindest unter 40 mmHg der Druckmanschette erzielen. Alternativ kann der zweite Beaufschlagungsbefehl zur Beaufschlagung der Manschette zu einem subsystolischen Blutdruck einen venösen Rückfluss-Stopp ermöglichen. Ein venöser Rückfluss-Stopp findet typischerweise in einer Druckbeaufschlagung der Manschette in einem Bereich von 40 - 60 mmHg statt.

Das Verfahren kann zudem umfassen Blutdruckmesswerte von der Manschette, insbesondere durch die Kommunikationsschnittstelle, zu empfangen. Somit kann ein Zeitintervall der Messung einfach und schnell gewählt werden.

Die Kontrolleinheit kann ein mobiles Endgerät sein, insbesondere ein Smartphone oder ein Tablet.

Die zeitlich aufeinanderfolgende Vielzahl von unterschiedlichen Messdaten können in einem vorbestimmten zeitlichen Abstand zueinander nach der Wiederherstellung des Blutflusses aufgenommen worden sein.

Die Vielzahl von Messdaten zu der Bestimmung der kardiovaskulären Leistung kann über ein Zeitintervall aus einem Bereich von 1 s bis 60 s, insbesondere aus einem Bereich von 1 s bis 35 s, vorzugsweise aus einem Bereich von 1 s bis 25 s, ausgewählt sein. Alternativ kann das Zeitintervall so gewählt sein, dass eine Abweichung von zeitlich direkt aufeinander folgenden Messdaten immer grösser ist als 5 %, insbesondere 4 %, vorzugsweise 3 %, ist. In einer weiteren Alternative kann das Zeitintervall so gewählt sein, dass ein Mittelwert von drei, vier, oder fünf zeitlich unmittelbar aufeinander folgenden gemessenen Messdaten grösser ist als ein vordefinierter Anteil, insbesondere 75 %, vorzugsweise 85 %, eines zeitlich unmittelbar darauffolgend gemessenen Messwertes.

In Abhängigkeit einer Pulsfrequenz, insbesondere gemessen durch den Sensor, kann eine diesem Zeitintervall entsprechende Anzahl an Messdaten, die zu der Anzahl der Pulse in diesem Zeitintervall korrespondieren, zur Bestimmung der kardiovaskulären Leistung ausgewählt werden.

Dies ermöglicht eine schnelle und einfache Bestimmung der kardiovaskulären Leistung des Patienten mit Mitteln, die in medizinischen Kreisen im Wesentlichen weit verbreitet verfügbar und leicht zugänglich sind.

Die Sättigung der Blutperfusion kann abhängig vom Patienten und körperlicher Belastung des Patienten unterschiedlich schnell erfolgen und/oder Überschwinger aufweisen. Insbesondere kann die Sättigung der Blutperfusion erst nach einem Einschwingverhalten in einem Einschwingbereich der Blutperfusion einsetzen. Das Einschwingverhalten ist abhängig von der kardiovaskulären Leistung als auch der Pulsfrequenz.

Es ist somit wünschenswert, wenn das Zeitintervall durch ein Abbruchkriterium, wie etwa der Abweichung von zeitlich aufeinander folgenden Messwerten, den Zeitintervall der Messung bestimmt.

Die Vielzahl der Messdaten können Messdaten umfassend oder daraus bestehen, die auf die Sauerstoffsättigung im Blut schliessen lassen, insbesondere SpO₂-Messdaten, volumetrische Messdaten, die auf die Perfusionsamplitude im Messabschnitt schliessen lassen, insbesondere Perfusionsindex-Messdaten, und/oder plethymographische Messdaten, die auf das Erkennen einer Pulsation schliessen lassen. Optional können die Messdaten zusätzlich Pulsfrequenzen umfassen.

Die Pulsfrequenz ist die Anzahl der Herzschläge pro Minute eines Patienten.

Diese Messdaten können mit einfachen und kostengünstigen Mitteln gemessen werden und sind in der klinischen Praxis verfügbar und leicht zugänglich.

Messdaten umfassend die Pulsfrequenz(en) sind vorteilhaft, da die kardiovaskuläre Leistung abhängig von der aktuellen Pulsfrequenz des Patienten ist. Zudem kann das Herzzeitvolumen aus dem Herzschlagvolumen und der Pulsfrequenz einfach bestimmt werden. Das Verfahren kann umfassen, dass als Zwischenschritt zur Bestimmung des Herzzeitvolumens ein Herzschlagvolumen bestimmt wird, welches mit der Pulsfrequenz multipliziert wird, um das Herzzeitvolumen zu bestimmen.

Die Pulsfrequenz kann von dem Sensor oder einem anderen Sensor, insbesondere einen anderen Sensor, der in der Manschette integriert ist, ermittelt werden und der Kontrolleinheit zur Verfügung gestellt werden.

Zusätzlich können patientenspezifische Daten der Kontrolleinheit zur Verfügung gestellt werden, die bei der Bestimmung der kardiovaskulären Leistung berücksichtigt werden, um diese zuverlässiger zu bestimmen. Die patientenspezifischen Daten können etwa einen Abstand der Manschette zum Messabschnitt betreffen, eine Grösse des Patienten, und/oder ein Gewicht des Patienten. Der Sensor und die Manschette können zudem konfiguriert sein den Abstand der Manschette zum Messabschnitt automatisch zu bestimmen.

Zudem können zwei oder mehr Sensoren, die an unterschiedlichen Bereichen der distalen Peripherie des Patienten verwendet werden, um während einer Messung einer Vielzahl von Messdaten zur Bestimmung der kardiovaskulären Leistung der Kontrolleinheit zur Verfügung zu stellen.

Zusätzlich zum Empfangen von Messdaten des Sensors an der distalen Peripherie, in der der Blutfluss unterbunden wurde, kann das Verfahren umfassen, eine Vielzahl von Messdaten eines weiteren Sensors zu empfangen, der an einer weiteren distalen Peripherie, in der der Blutfluss nicht unterbunden wurde/wird.

Eine Perfusionsamplitude, und insbesondere eine Herzschlagvolumenvariabilität, kann aus der Vielzahl der Messdaten bestimmt werden, insbesondere aus den SpO₂-Messdaten, den Perfusionsindex-Messdaten, und/oder den plethysmographischen Messdaten.

Die Bestimmung einer Perfusionsamplitude für jeden Messwert der Messdaten ermöglicht eine Änderung der Perfusion zu messen.

Die Herzschlagvolumenvariabilität kann sich direkt aus dem plethysmographischen Messdaten ergeben. Die Herzschlagvolumenvariabilität kann sich etwa durch eine Abweichung des gemessenen Perfusionsindex gegenüber einem Maximalwert des Perfusionsindex im Zeitintervall oder Durchschnittswert des Perfusionsindex im Sättigungsbereich bestimmt werden.

Die Herzschlagvolumenvariabilität könnte auch durch eine Abweichung eines Pulsdrucks gegenüber einem Maximalwert oder Durchschnittswert des Pulsdrucks bestimmt werden.

Die Herzschlagvolumenvariabilität kann vorzugsweise für jeden einzelnen Sensorpuls individuell bestimmt werden.

Besonders im Bereich eines steilen Anstiegs der Werte der Messdaten nach dem Ermöglichen des Blutflusses zu der distalen Peripherie ist der Einfluss der Herzschlagvolumenvariabilität besonders gross, während der Einfluss später im Einschwing-/Sättigungsbereich stark abnimmt. Es ist somit wünschenswert besonders im Bereich des steilen Anstiegs die Herzschlagvolumenvariabilität zu berücksichtigen, um die kardiovaskuläre Leistung zu bestimmen. Die Messdaten, etwa Perfusionsamplituden, steigen bereits in kurzen Zeiträumen von wenigen Sekunden stark an, sodass kleine Abweichungen ansonsten zu relativ hohen Messungenauigkeiten führen könnten.

Besonders für hohe Pulsfrequenzen kann dies besonders problematisch sein, da eine Messfrequenz des Sensors oftmals geringer ist als die Pulsfrequenz.

Die Herzschlagvolumenvariabilität kann verwendet werden, um die Messdaten, insbesondere die Perfusionsamplitude, zu korrigieren. Somit ist es möglich für eine Schwankung des Herzschlagvolumens durch Berücksichtigung der Herzschlagvolumenvariabilität zu korrigiert, welche unter anderem durch den respiratorischen Rhythmus, Herzrhythmusstörungen, pathologische Zustände, Blutvolumenstatus des Patienten, thorakalen Druck, Körperlage, und/oder Bewegung beeinflusst werden kann.

Die Perfusionsamplitude kann normiert sein. So kann etwa ein Messwert mit dem maximalen Wert des Messwerts im Zeitintervall, einen Mittelwert im Einschwingbereich/Sättigungsbereich der Messdaten, oder den letzten Messwert des Zeitintervalls normiert sein. So kann etwa der gemessene Perfusionsindex durch einen maximalen Perfusionsindex im Zeitintervall normiert werden. Die Messung der Perfusionsamplitude ist also ein relativer Messwert.

Die Perfusionsamplitude kann aus einem Verhältnis einer pulsatilen Komponente, die Aufschluss über eine Änderung der Lichtabsorption und somit über die Blutperfusion liefert, und einer nicht pulsatilen Komponente, die Aufschluss über die nicht pulsatilen Elemente, wie Haut, Knochen und venöses Blut liefert, bestimmt werden.

Das computerimplementierte Verfahren kann eine Bestimmung der kardiovaskulären Leistung mit Hilfe zumindest einer Fit-Funktion der Perfusionsamplitude umfassen. Die zumindest eine Fit-Funktion kann eine polynomische Fit-Funktion, eine logarithmische Fit-Funktion, eine exponentielle Fit-Funktion, insbesondere mit schwingender Dämpfung, eine logistische Wachstumsmodellfit-Funktion, insbesondere mit schwingender Dämpfung, eine Potenzgesetzsättigungsfit-Funktion, eine Hill Fit-Funktion, eine Differentialgleichungsfit-Funktion, insbesondere zweiter Ordnung, umfassen oder daraus bestehen.

Das computerimplementierte Verfahren kann eine Bestimmung der kardiovaskulären Leistung mit Hilfe eines Algorithmus umfassend eine Bereitstellung einer Vielzahl von Referenz-Funktionen, die vorzugsweise zeitlich vorher gemessene Messdaten umfassen, insbesondere zeitlich vorher gemessene Messdaten einer ähnlichen oder der gleichen Pulsfrequenz. Dieser Algorithmus umfasst zudem eine Bestimmung einer Abweichung der Vielzahl von Referenz-Funktionen mit der Vielzahl von empfangenen Messdaten oder von Korrelationswerten der Messdaten, und eine Auswahl einer Referenz-Funktion der Vielzahl von Referenz-Funktionen mit der geringsten Abweichung oder eines ähnlichsten Korrelationswerts zu den empfangenen Messdaten.

Die Bestimmung der kardiovaskulären Leistung kann mit Hilfe einer über ein Zeitintervall der Messung gemittelten Perfusionsamplitude, welche mit der Pulsfrequenz verrechnet wird, insbesondere vollständig oder anteilig mit der Pulsfrequenz multipliziert wird erfolgen.

Die Bestimmung der kardiovaskulären Leistung kann mit Hilfe eines maschinellen Lernmodells, insbesondere eines vortrainierten maschinellen Lernmodells, anhand der Perfusionsamplituden erfolgen.

Diese zumindest eine Fit-Funktion ermöglicht einen zuverlässigen Fit üblicher Werte der Perfusionsamplitude. Insbesondere kann die Fit-Funktion so gewählt sein, dass ein zuverlässiger Fit der Werte der Perfusionsamplitude ermöglicht wird, die über einen Sättigungsbereich überschwingen bevor die Werte der Perfusionsamplitude lediglich in dem Sättigungsbereich schwanken. Zudem kann die Fit-Funktion gewählt sein, um abzubilden, dass die Perfusionsamplitude in dem Einschwingbereich zumindest ein lokales Maximum erreicht, daraufhin wieder abfällt und schliesslich zum Sättigungsbereich ansteigt, um lediglich in dem Sättigungsbereich zu schwanken. Dies kann etwa durch Polynome höherer Ordnung als Fit-Funktion erfolgen.

Die Bestimmung der kardiovaskulären Leistung mit Hilfe einer gemittelten Perfusionsamplitude oder eines maschinellen Lernmodells ermöglicht eine zuverlässige und einfache Approximation der kardiovaskulären Leistung.

Eine ähnliche Pulsfrequenz ist in diesem Zusammenhang eine Pulsfrequenz, die in weniger als 5 Schläge pro Minute, vorzugsweise weniger als 3 Schläge pro Minute, vorzugsweise weniger als 2 Schläge pro Minute, von der Pulsfrequenz des Patienten abweicht. Bevorzugt kann eine ähnliche Pulsfrequenz weniger als 1 Schlag pro Minute abweichen.

Der Korrelationswert kann etwa ein Korrelationskoeffizient sein, insbesondere etwa der Spearman-Korrelationskoeffizient.

Das computerimplementierte Verfahren kann umfassen, dass eine Fit-funktion oder mehrere Fit-Funktionen der Perfusionsamplitude aus einer Vielzahl von verschiedenen Fit-Funktionen ausgewählt wird und/oder kombiniert werden.

Als Metrik zur Auswahl der Fit-Funktion oder Referenzfunktion kann etwa eine Abweichung, insbesondere der mittlere absolute Fehler oder mittlerer quadratischer Fehler/Wurzelfehler, der Fit-Funktion oder Referenzfunktion mit der Perfusionsamplitude der Messdaten bestimmt werden. Die Metrik kann zudem gewichtet werden, insbesondere gewichtet werden, um den Bereich des stärksten Anstiegs der Perfusionsamplitude besonders zu berücksichtigen und eine Sättigung nach wiederhergestelltem Blutfluss weniger stark zu berücksichtigen. Insbesondere kann die Abweichung innerhalb der ersten 10 s, insbesondere der ersten 8 s, vorzugsweise der ersten 4 s, der Zeitintervall der Messung besonders stark gewichtet werden.

Es kann eine Summe / Integration, über die Perfusionsamplitude, die Referenzfunktion, und/oder die Fit-Funktion(en) bis zum Erreichen einer Sättigung der Perfusionsamplitude dazu verwendet werden, die kardiovaskuläre Leistung zu bestimmen.

Die Messwerte an sich geben lediglich eine Stufenfunktion an, da nur an diskreten Stellen Messwerte vorhanden sind, sodass diese einer gewissen Ungenauigkeit unterliegen. Eine Integration der Fit-Funktion(en) kann hingegen ebenfalls diesen Übergang zwischen den einzelnen Messwerten berücksichtigen.

Die Fitfunktion mit der geringsten Abweichung kann ausgewählt werden.

Die Steigung, also die erste Ableitung, der besten Fit-Funktion kann an bestimmten Zeitpunkten bestimmt werden, um die kardiovaskuläre Leistung, insbesondere das Herzzeitvolumen zu bestimmen. Die Messung der Steigung kann bei einem Zeitpunkt erfolgen, der ein Überschreiten eines vorbestimmten Anteils bis zu einem Sättigungswert darstellt.

Das Verfahren kann umfassen, dass mehrere Fitfunktionen unabhängig voneinander verwendet werden, um das Herzzeitvolumen zu bestimmen.

Das Verfahren kann umfassen, dass eine Summe der normierten Perfusionsamplitude der Vielzahl von Messdaten in einem Zeitintervall gemittelt wird.

Das Zeitintervall der Mittelung kann anhand (i) eines speziellen Algorithmus, insbesondere einer Fit-Funktion, (ii) anhand wiederholter Messung von Messwerten, die nicht signifikant voneinander abweichen, oder (iii) anhand eines Zeitpunktes einer Überschreitung von einem vorbestimmten Anteil, insbesondere etwa 66 %, der maximalen Perfusionsamplitude oder einem Sättigungswert der Perfusionsamplitude bestimmt werden.

Für eine exaktere Messung kann zudem der erste gemessene Wert der normierten Perfusionsamplitude von der Summe subtrahiert werden.

Der gemittelte Wert der Perfusionsamplituden im Zeitintervall der Messung, oder ein vordefinierter Anteil davon, kann mit der Pulsfrequenz multipliziert wird um das Schlagvolumen näherungsweise zu bestimmen. Durch erneute Multiplikation mit der Pulsfrequenz kann das Herzzeitvolumen näherungsweise bestimmt werden.

Der Abstand zwischen dem ersten Beaufschlagungsbefehl und dem zweiten Beaufschlagungsbefehl kann mindestens 15 s, insbesondere mindestens 20 s, vorzugsweise mindestens 25 s betragen. Der zeitliche Abstand kann so gewählt sein, sodass ein Messwert oder ein Mittelwert von 2, 3, 4, oder 5 zeitlich unmittelbar aufeinander folgenden gemessenen Messdaten, insbesondere Perfusionsindex-Messdaten, kleiner ist als ein vordefinierter Anteil, insbesondere 20 %, vorzugsweise 15 %, eines gemessenen Messwertes oder Mittelwerts von 2, 3, 4, oder 5 zeitlich unmittelbar aufeinanderfolgenden Messwerten, der vor der Druckbeaufschlagung gemessen wurde.

Ein solcher erster Beaufschlagungsbefehl kann sicherstellen, dass die Perfusion von Blut zuverlässig unterbunden wurde, bevor die Messung nach Wiederherstellung des Blutflusses beginnt. Dies ermöglicht insbesondere für SpO₂-Messwerte eine optimierte Messung, da durch eine solche Verzögerung der Freigabe des Blutflusses weniger Hämoglobin Sauerstoff gebunden hat, also die Sauerstoffsättigung abgenommen hat.

Alternativ kann der zweite Beaufschlagungsbefehl gesendet werden nachdem der systolische Blutdruck durch die Druckbeaufschlagung Manschette für einen vordefinierten Zeitraum überschritten wurde. Der Zeitraum kann ausgewählt sein aus 2 s - 12 s, insbesondere 4 s - 10 s, vorzugsweise 5 s - 9 s. Insbesondere kann der Zeitraum 3 s, 4 s, 5 s, 6 s, 7 s, 8 s, 9 s, 10 s, oder 11 s betragen.

Die Vielzahl von Messdaten des Sensors können in Ruhe und/oder unter körperlicher Belastung des Patienten detektiert werden.

Eine solche Vielzahl von Messdaten ermöglicht eine zuverlässigere Bestimmung der kardiovaskulären Leistung(en), da die Vielzahl an Messdaten in einer kontrollierten Belastung detektiert werden. Zudem ist die Detektion der Vielzahl von Messdaten mit den vorhergehend beschriebenen Messdaten, insbesondere den SpO₂-Messdaten, auch bei körperlicher Belastung einfach möglich, da diese durch kompakte und tragbare Sensoren ermittelt werden können.

Die kardiovaskuläre Leistung schwankt erheblich, so kann etwa ein Herzzeitvolumen eines Erwachsenen in Ruhe etwa 4,5 L/min - 5,5 L/min, während es bei körperlicher Belastung etwa auf 15 L/min - 25 L/min ansteigt. Somit kann insbesondere durch eine Vielzahl von Messdaten, welche in Ruhe und unter körperlicher Belastung detektiert wurden, die Messgenauigkeit erhöht werden. Die körperliche Belastung kann einen vordefinierten Wert entsprechen, etwa einem üblichen Ergometrie-Test entsprechen, insbesondere einem Ergometrie-Test mit dem Fahrrad oder eines Bruce Protokoll Ergometrie-Test. Insbesondere kann die körperliche Belastung einen Wert von 50 W - 450 W, insbesondere 100 W - 200 W, vorzugsweise 125 W - 175 W entsprechen.

Das computerimplementierte Verfahren kann eine erste Bestimmung einer ersten kardiovaskulären Leistung anhand von Messdaten, die in Ruhe aufgenommen wurden und eine zweite Bestimmung einer zweiten kardiovaskulären Leistung anhand von Messdaten, die unter körperlicher Belastung des Patienten aufgenommen wurden, umfassen.

Eine solche Bestimmung der ersten und zweiten kardiovaskulären Leistung ermöglicht eine zuverlässigere Einschätzung der kardiovaskulären Gesundheit des Patienten. Insbesondere können mögliche Erkrankungen wie Fieber, Hyperthyreose, Hypertonie, Herzinsuffizienz, Kreislaufschock, Aortenklappenstenose, oder koronare Herzkrankheit, direkt oder indirekt die kardiovaskuläre Leistung beeinflussen.

Ein weiterer Aspekt der Erfindung richtet sich auf ein Computerprogrammprodukt mit Anweisungen, die, wenn das Programm von einem Computer ausgeführt wird, den Computer veranlassen, das zuvor beschriebene computerimplementierte Verfahren auszuführen.

Ein weiterer Aspekt der Erfindung richtet sich auf ein computerlesbares Medium, das Anweisungen enthält, die, wenn sie von einem Computer ausgeführt werden, den Computer veranlassen, das zuvor beschriebene computerimplementierte Verfahren auszuführen.

Ein weiterer Aspekt der Erfindung richtet sich auf ein System, welches zum Durchführen des zuvor beschriebenen computerimplementierten Verfahrens, umfassend Mittel, insbesondere eine Kommunikationsschnittstelle, eine Ausgangsschnittstelle, und eine Kontrolleinheit, zum Ausführen des computerimplementierten Verfahrens.

Dies ermöglicht eine integrale Lösung eines Systems zum Durchführen des zuvor beschriebenen Verfahrens zur Verfügung zu stellen, den Implementierungsaufwand zu reduzieren, die Kompatibilität und die Funktionalität der Komponenten zu optimieren, und eine verbesserte Benutzererfahrung zu ermöglichen.

Das System kann zumindest einen Sensor, der mit der Kontrolleinheit verbunden oder verbindbar ist, für die distale Peripherie eines Patienten umfassen. Der zumindest eine Sensor kann zum Messen einer Vielzahl von Messdaten, die auf die Sauerstoffsättigung im Blut schliessen lassen, konfiguriert sein. Der zumindest eine Sensor kann einen Sensor zum Messen von volumetrischen Messdaten, plethysmographischen Messdaten, und optional Pulsfrequenzen umfassen oder daraus bestehen.

Solche Sensoren, insbesondere ein SpO₂-Sensor, sind kompakt und leicht in Technologie, insbesondere tragbare Technologie, integrierbar und kostengünstig. Insbesondere sind solche Sensoren kompakter und leichter in der Handhabung als etwa ein Doppler-Ultraschallmessgerät, welches ebenfalls für eine Blutperfusionsmessung verwendet werden könnte.

Der zumindest eine Sensor kann ein Fingerpulsoximeter, ein Handgelenkspulsoximeter, eine tragbare Uhr, insbesondere eine Smartwatch, oder ein Fitnessarmband mit integriertem Pulsoximetersensor, ein stationäres Pulsoximetergerät mit abnehmbaren Sensoren, und/oder ein Hautpflasterpulsoximeter umfassen oder daraus bestehen.

Solche Sensoren sind besonders einfach und/oder praktikabel in der Handhabung, benutzerfreundlich, und weisen insbesondere eine geringe Dimensionierung auf.

Der zumindest eine Sensor kann durch einen elektrischen Leiter oder eine drahtlose Verbindung mit der Kontrolleinheit verbunden oder verbindbar sein. Zudem kann der Sensor durch einen elektrischen Leiter oder eine drahtlose Verbindung mit der Manschette verbunden sein.

Das System kann eine Manschette umfassen. Die Manschette ist durch einen Beaufschlagungsbefehl der Kontrolleinheit mit Druck beaufschlagbar, um den Blutfluss zu einer distalen Peripherie des Patienten zu unterbinden und wiederherzustellen.

Dies ermöglicht eine optimierte Interoperabilität der Manschette, des zumindest einen Sensors und der Kontrolleinheit und eine automatisierte und/oder synchronisierte Durchführung des Verfahrens mit reduzierten Aufwand des Nutzers.

Das System kann eine Benutzeroberfläche, insbesondere umfassend zumindest einen oder einen Touchscreen und/oder Bedienelemente, für eine Benutzereingabe umfassen. Das System kann dazu konfiguriert sein das zuvor beschriebene computerimplementierte Verfahren voll- oder teilautomatisch auszuführen, insbesondere auf Basis der Benutzereingabe.

Die Manschette kann durch einen elektrischen Leiter oder eine drahtlose Verbindung mit der Kontrolleinheit verbunden oder verbindbar sein.

Die Manschette kann einen Sensor zum Detektieren von Messdaten umfassend die Pulsfrequenz des Patienten inkludieren und diese Messdaten der Kontrolleinheit zur Verfügung zu stellen.

Die Manschette kann konfiguriert sein, um einen systolischen Blutdruck des Patienten beim Beaufschlagen mit Druck zu bestimmen, und optional einer Kontrolleinheit zur Verfügung stellen.

Ein weiterer Aspekt der Erfindung richtet sich auf ein Verfahren zur Bestimmung der kardiovaskulären Leistung aus einer Vielzahl von Messdaten. Das Verfahren umfassen eine Beaufschlagung einer Manschette über einen systolischen Blutdruck eines Patienten, um einen Blutfluss zu einer distalen Peripherie des Patienten zu unterbinden. Das Verfahren inkludiert eine Beaufschlagung der Manschette zu einem subsystolischen Blutdruck, um einen Blutfluss zu der distalen Peripherie zu ermöglichen. Das Verfahren umfasst Messen einer Vielzahl von Messdaten von einem Sensor an einem Messabschnitt der distalen Peripherie des Patienten. Die Messdaten geben Aufschluss über eine Perfusion am Messabschnitt. Die Vielzahl der Messdaten umfassen zumindest zwei verschieden Messdaten, die zu unterschiedlichen Zeiten nach dem Wiederherstellen des Blutflusses zu der distalen Peripherie detektiert wurden. Das Verfahren umfasst zudem die Bestimmung der kardiovaskulären Leistung, insbesondere des Herzzeitvolumens, anhand der Vielzahl von Messdaten.

Ein weiterer Aspekt der Erfindung betrifft ein computerimplementiertes Verfahren zum Trainieren eines maschinellen Lernmodells für die Bestimmung einer kardiovaskulären Leistung, insbesondere des zuvor beschriebenen maschinellen Lernmodells. Das computerimplementierte Verfahren umfasst das Empfangen eines Eingabetrainingsdatensatzes, der Messdaten und einen zugehörigen Zielwert einer kardiovaskulären Leistung umfasst. Die Messdaten wurden von zumindest einem Sensor an einem Messabschnitt einer distalen Peripherie eines Patienten nach dem Wiederherstellen eines Blutflusses der distalen Peripherie detektiert. Die Messdaten sind vorzugsweise zuvor beschriebene Messdaten. Das computerimplementierte Verfahren inkludiert das Durchführen eines Trainingsprozesses des maschinellen Lernmodells durch Vorhersagen eines Ausgabewerts, Vergleichen des Ausgabewerts mit dem Zielwert, Anpassen der Modellparameter durch eine Verlustfunktion. Dieser Trainingsprozess wird iterativ für weitere Eingabetrainingsdatensätze bis zu einem Abbruchkriterium wiederholt. Vorzugsweise wird das trainierte Modell validiert durch Anwendung auf einen separaten Validierungsdatensatz umfassend Validierungsmessdaten und korrespondierende kardiovaskuläre Validierungsleistungen zum Überprüfen des Modells. Zudem wird das trainierte Modell bereitgestellt.

Ein weiterer Aspekt der Erfindung richtet sich auf den Trainingsdatensatz/Validierungsmessdatensatz zur Verwendung in dem zuvor beschriebenen computerimplementierten Verfahren zum Trainieren eines maschinellen Lernmodells umfassend zumindest einen Eingabetrainingsdatensatz.

Der Eingabetrainingsdatensatz kann auf die Sauerstoffsättigung im Blut schliessen lassen, insbesondere Messdaten umfassen, die auf die Sauerstoffsättigung im Blut schliessen lassen, vorzugsweise SpO₂-Messdaten, volumetrische Messdaten, plethysmographische Messdaten, und vorzugsweise Pulsfrequenzen, umfassen oder daraus bestehen.

Weitere Ausführungsformen der Erfindung und Verbesserungen der beschriebenen Ausführungsformen werden in der folgenden Beschreibung der Ausführungsformen deutlich.

Die Erfindung wird nun unter Bezugnahme auf bestimmte Ausführungsformen und Figuren beschrieben, die zeigen:
- Figur 1:: eine perspektivische Ansicht eines erfindungsgemässen Systems zur Bestimmung der kardiovaskulären Leistung gemäss einem erfindungsgemässen computerimplementierten Verfahren,
- Figur 2:: eine schematische Darstellung des erfindungsgemässen computerimplementierten Verfahrens,
- Figur 3:: eine Vielzahl von Perfusionsamplituden der Messdaten im Zeitverlauf, die in Ruhe des Patienten ermittelt wurden und durch eine polynomische Fit-Funktion approximiert wurden,
- Figur 4:: eine Vielzahl von Perfusionsamplituden der Messdaten im Zeitverlauf, die in Ruhe des Patienten ermittelt wurden und einen Überschwinger aufweisen,
- Figur 5:: eine Vielzahl von Perfusionsamplituden der Messdaten im Zeitverlauf, die bei körperlicher Belastung des Patienten ermittelt und durch eine logarithmische Fit-Funktion approximiert wurden, und
- Figur 6:: einen Trainingsdatensatz im Zeitverlauf, exemplarisch umfassend fünf Messreihen von Perfusionsamplituden, zum Trainieren des maschinellen Lernmodells.

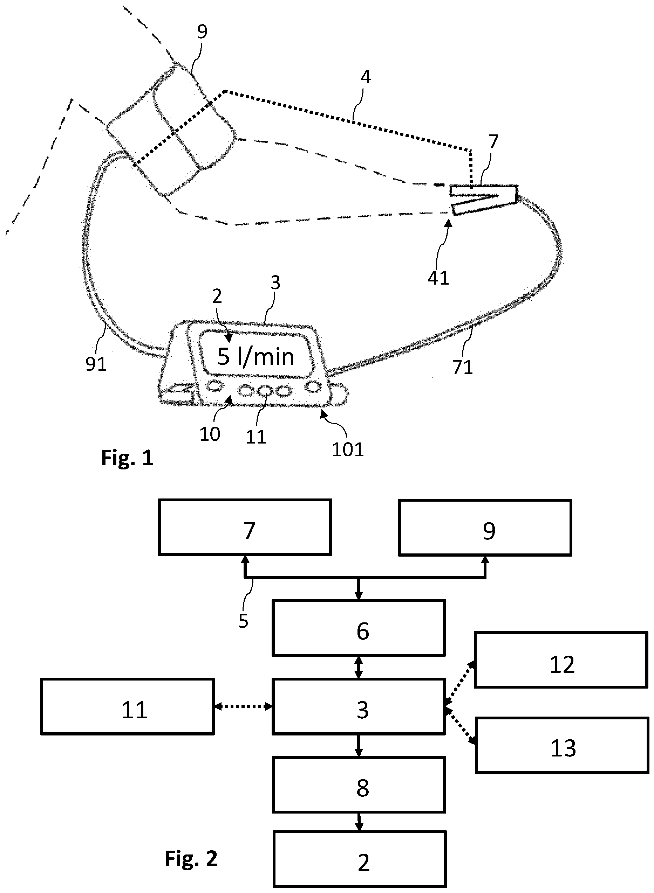

Die Figur 1 zeigt eine perspektivische Ansicht eines erfindungsgemässen Systems 101 zu Bestimmung der kardiovaskulären Leistung in Form des Herzzeitvolumens gemäss einem erfindungsgemässen computerimplementierten Verfahren. Das System 101 umfasst eine Kontrolleinheit 3, die über Kabel 71, 91 mit einer Manschette 9 und einem SpO₂-Sensor 7 verbunden ist. Alternativ kann die Manschette 9 und/oder der SpO₂-Sensor 7 kabellos mit der Kontrolleinheit 3 verbunden sein. Der SpO₂-Sensor 7 ist ein Fingerpulsoximeter, welches an einem Finger einer distalen Peripherie 4 eines Patienten angebracht werden kann, um die Sauerstoffsättigung im Blut an einem Messabschnitt 41 zu bestimmen. Es können jedoch andere Sensoren 7 verwendet werden, die dazu geeignet sind eine Blutperfusion zu messen.

Die distale Peripherie 4 in Form einer oberen Extremität des Patienten und ein Teil einer Kontur des Patienten sind durch eine gestrichelte Linie in Fig. 1 gekennzeichnet.

Die Kontrolleinheit 3 ist mit einer Benutzeroberfläche 10 verbunden, die Bedienelemente 11 in Form von mechanischen Knöpfen umfasst. Durch diese Bedienelemente 10 kann ein Nutzer des Systems 101 den Messvorgang starten.

Wenn der Messvorgang gestartet wurde, wird die Messung vorzugsweise vollautomatisiert ausgeführt. Die Kontrolleinheit 3 sendet über eine Kommunikationsschnittstelle 6 (siehe Fig. 2) einen ersten Beaufschlagungsbefehl an die Manschette 9, sodass diese über den systolischen Blutdruck eines Patienten mit Druck beaufschlagt wird, sodass der Blutfluss zu der Peripherie 4 des Patienten unterbunden wird.

Anschliessend sendet die Kontrolleinheit 3 über eine Kommunikationsschnittstelle einen zweiten Beaufschlagungsbefehl, sodass diese zu einem subsystolischen Blutdruck beaufschlagt wird, sodass ein Blutfluss zu der distalen Peripherie ermöglicht 4 wird, und insbesondere ebenfalls ein venöser Rückfluss ermöglicht wird.

Der zeitliche Abstand zwischen dem ersten und zweiten Beaufschlagungsbefehl sind etwa so gewählt, dass ein Mittelwert von vier zeitlich unmittelbar aufeinander folgenden Perfusionsindex-Messwerten kleiner ist als 20 % eines Perfusionsindex-Messwertes der zeitlich unmittelbar auf diese Perfusionsindex-Messwerte folgt.

Alternativ zu einer Steuerung der Manschette 9 über die Kontrolleinheit 3 kann die Manschette 9 auch unabhängig von dem System 101, etwa durch einen Nutzer manuell oder eine weitere Steuerung betrieben werden.

Sobald der Blutfluss zu der distalen Peripherie 4 durch die Manschette 9 freigegeben wurde, wird eine Vielzahl von Perfusionsindex-Messwerte durch den SpO₂-Sensor 7 detektiert. Ein Zeitintervall der Messung wird zum Beispiel über 60 s ausgeführt. Dieses Zeitintervall ist so gewählt, dass ein Mittelwert von vier zeitlich unmittelbar aufeinander folgenden Messdaten grösser ist als 85 % des darauffolgenden Messwerts.

Anschliessend wird eine Perfusionsamplitude, also etwa ein auf einen Maximalwert normierter Perfusionsindex, für die Messdaten bestimmt. Die Perfusionsamplitude kann normiert sein, indem etwa die gemessenen Perfusionsindex-Messwerte durch einen Maximalwert im Messintervall, einen Endwert im Messintervall, oder einen gemittelten Sättigungswert des Messintervalls geteilt wird. Der gemittelte Sättigungswert kann sich aus einer Mittelung aller Messwerte eines Sättigungsbereichs ergeben, indem die Messwerte lediglich in einem gewissen Bereich variieren.

Die Kontrolleinheit 3 ist konfiguriert die anhand von nachfolgend beschriebenen Verfahren die kardiovaskuläre Leistung 2 in Form des Herzzeitvolumens durch diese Perfusionsamplitudenwerte zu bestimmen (siehe Fig. 2).

Anschliessend wird das Herzzeitvolumen, das beispielsweise 5 l/min betragen kann, durch eine Ausgangsschnittstelle 8 (siehe Fig. 2) bereitgestellt und wie in Fig. 1 gezeigt auf einem Display ausgegeben.

Das computerimplementierte Verfahren könnte jedoch auch durch ein mobiles Endgerät wie ein Smartphone als eine Kontrolleinheit 3 durchgeführt werden, etwa mit einer Softwareanwendung für mobile Endgeräte. Zu diesem Zweck, könnte die Manschette 9 und/oder der Sensor 7 eine Datenübertragung im ISM-Frequenzbereich, vorzugsweise im Bereich von 2,4 GHz, zur Kommunikation mit dem mobilen Endgerät aufweisen.

Die Figur 2 zeigt eine schematische Darstellung des erfindungsgemässen computerimplementierten Verfahrens. Figure 2 zeigt einen Sensor 7 zur Aufnahme von SpO₂-Messdaten 5, volumetrische Messdaten, und/oder plethsymographische Messdaten, und optional einer Pulsfrequenz, und eine Manschette 9 zur Beaufschlagung mit Druck, um den Blutfluss zu einer distalen Peripherie eines Patienten zu unterbinden. Der Sensor 7 und die Manschette 9 sind uni- oder bidirektional mit einer Kommunikationsschnittstelle 6 verbunden. Diese Messdaten 5 werden von der Kontrolleinheit 3 in eine Perfusionsamplitude umgerechnet. Die Kontrolleinheit 3 kann über die Kommunikationsschnittstelle 6 sowohl Beaufschlagungsbefehle an die Manschette 9 senden und vorzugsweise Messdaten, insbesondere eine Pulsfrequenz und/oder einen aktuellen Druckbeaufschlagungswert empfangen. Somit kann sichergestellt werden, dass die Messung erst beginnt, wenn eine Beaufschlagung der Manschette 9 mit einem Zieldruck erreicht wurde, also ein Druck über einem systolischen Blutdruck des Patienten. Zudem kann die Kontrolleinheit 3 eine Vielzahl an Messdaten 5, die Aufschluss

über eine Perfusion des Blutflusses an einem Messabschnitt liefern, von dem Sensor 7 über die Kommunikationsschnittstelle 6 empfangen.

Die Kontrolleinheit 3 kann mit Hilfe einer Fit-Funktion 11, eines maschinellen Lernmodells 12, eines deterministischen Näherungsverfahren, und/oder einer Referenzfunktion 13 die kardiovaskuläre Leistung 2 anhand der Messdaten 5 eines Patienten bestimmen, was durch gestrichelte Linien in Fig. 2 dargestellt wurde.

Zudem kann eine Herzschlagvolumenvariabilität für jeden Messwert der Messdaten 5 bestimmt werden oder erhalten werden. Die Messdaten 5 können anhand der Herzschlagvolumenvariabilität angepasst werden, um Schwankung des Herzschlagvolumens zu berücksichtigen.

Insbesondere kann durch die Herzschlagvolumenvariabilität ein Messwert antiproportional zur Schwankung korrigiert werden. Wenn ein Herzschlag ungewöhnlich wenig/viel Volumen gepumpt hat und ein Herzschlag ansonsten typischerweise mehr/weniger Blut pumpt, kann dieser nach oben/unten korrigiert werden.

Anschliessend wird die kardiovaskuläre Leistung 2 durch eine Ausgangsschnittstelle 8 bereitgestellt. Die Ausgangsschnittstelle 8 kann eine Schnittstelle für eine Übergabe der kardiovaskuläre Leistung 2 oder ein Display zur Ausgabe der kardiovaskulären Leistung umfassen.

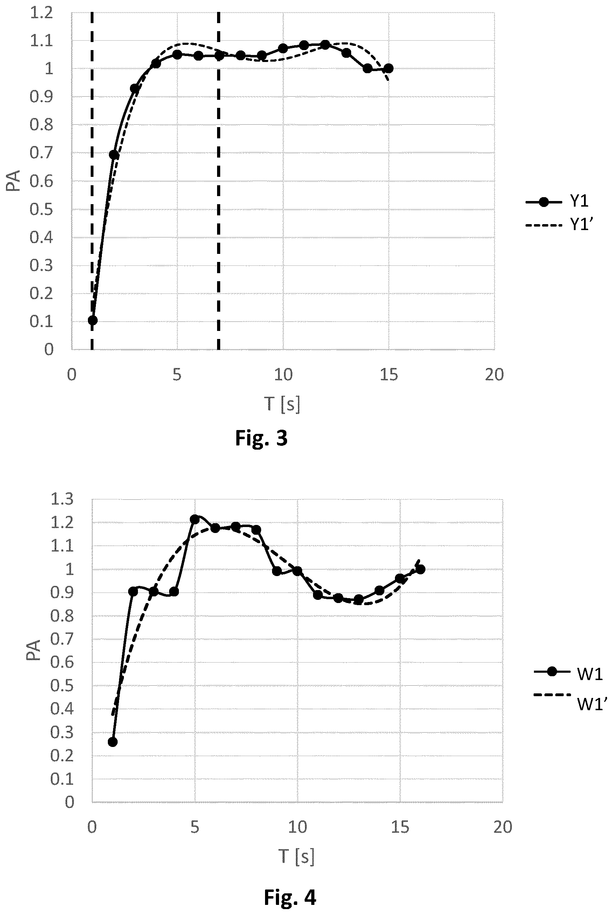

Figur 3 zeigt eine Vielzahl von Perfusionsamplituden der Messdaten Y1 im Zeitverlauf, die in Ruhe des Patienten ermittelt wurden, also bei einem Ruhepuls des Patienten ohne eine körperliche Belastung. Die Messdaten Y1 wurden unmittelbar nach der Ermöglichung des Blutflusses zu der distalen Peripherie gemessen. Ein Wert der Perfusionsamplitude wurde ermittelt, indem der mit einem Sensor gemessene Perfusionsindex-Messwert mit einem Messwert, der nach 15 Sekunden gemessen wurde, normiert wurde. Somit ergibt sich ein Verlauf der Messdaten Y1, der steil ansteigt und bereits nach wenigen Sekunden einen Sättigungsbereich erreicht und lediglich geringfügig in diesem Sättigungsbereich schwankt. Die Fit-Funktion Y1' aus Fig. 3 kann die Gesamtmessung von 15 Sekunden vorgenommen werden. Die Messdaten Y1 in Fig. 3 wurden durch eine Polynom-Fitfunktion Y1' vierter Ordnung gefittet, sodass eine stetige Funktion für die Ermittlung der kardiovaskulären Leistung verwendet werden kann.

Der Zeitintervall für eine Fit-Funktion Y1' in Fig. 3 kann jedoch angepasst werden, sodass Perfusionsamplituden im Sättigungsbereich, die natürlichen Schwankungen der Messwerte unterliegen, nicht übermässig berücksichtigt werden. Der Zeitintervall in Fig. 3 zum Ermitteln der Fit-Funktion Y1' kann etwa auf den gestrichelt umrahmten Bereich aus Fig. 3 eingeschränkt werden. Dies kann erreicht werden, indem der Zeitintervall etwa so gewählt wird, dass vier unmittelbar aufeinander folgenden Perfusionsamplituden Messwerte unter einem vordefinierten Anteil des darauffolgenden Perfusionsamplitudenwert liegen, etwa bei einem vordefinierten Anteil von 95 % des darauffolgenden Perfusionsamplitudenwert. Der Einschwingbereich aus Fig. 3 überlappt also mit dem Sättigungsbereich, i.e. nach einem Einschwingen wird direkt eine Sättigungswert angenommen, indem die Messdaten nur noch geringfügig um einen Sättigungswert schwanken. Die Messdaten, wie etwa Perfusionsamplitudendaten, können jedoch, insbesondere für grosse kardiovaskuläre Leistungen und/oder falls die Messdaten bei körperlicher Aktivität aufgenommen wurden, in dem Einschwingbereich oder nach dem Einschwingbereich erneut steigen, bis schliesslich ein Sättigungsbereich erreicht wird. Der Einschwingbereich kann einen oder mehrere Überschwinger aufweisen (siehe Fig. 4).

Anhand einer solchen Fit-Funktion Y1' kann die Kontrolleinheit die kardiovaskuläre Leistung ermitteln.

Figur 4 zeigt eine Vielzahl von Perfusionsamplituden der Messdaten W1 im Zeitverlauf, die in Ruhe des Patienten ermittelt wurden und einen Überschwinger aufweisen. Die Messdaten W1 wurden exemplarisch ebenfalls mit einer Polynom-Fitfunktion W1' gefittet, um die kardiovaskuläre Leistung zu ermitteln.

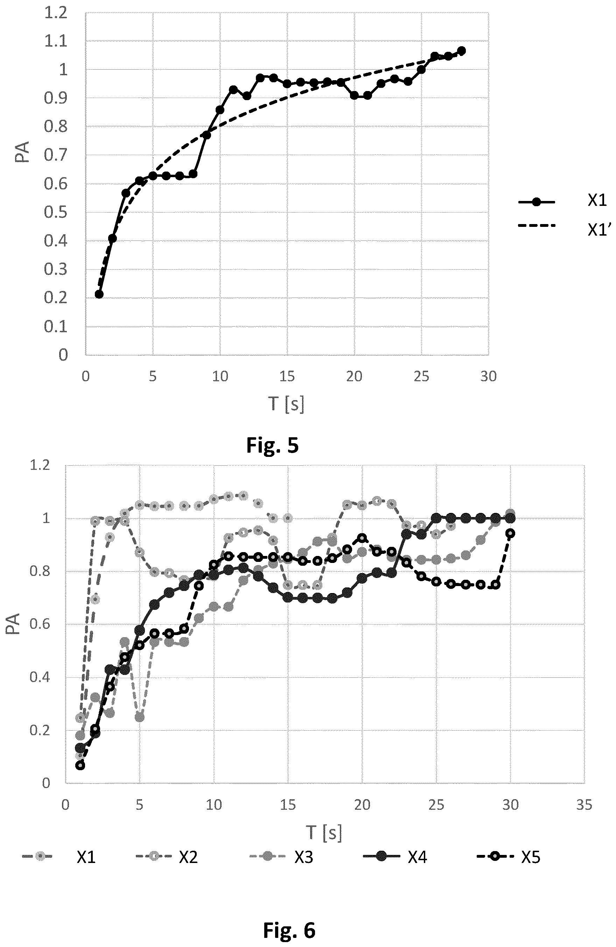

Figur 5 zeigt eine Vielzahl von Perfusionsamplituden der Messdaten X1 im Zeitverlauf, die bei körperlicher Belastung von 150 W des Patienten ermittelt. Die Perfusionsamplituden im Zeitverlauf wurden durch eine logarithmische Fit-Funktion X1' approximiert. Figur 5 zeigt zudem, dass ein deutlich längeres Zeitintervall notwendig ist, um einen Sättigungsbereich der Perfusionsamplitude zu erreichen. Für einen solchen Fall kann eine andere Fit-Funktion eine logarithmische Fit-Funktion eine bessere Approximation der kardiovaskulären Leistung ermöglichen. Da die kardiovaskuläre Leistung bei körperlicher Belastung deutlich höher ist, nimmt es mehr Zeit in Anspruch bis die Perfusionsamplitude einen Sättigungsbereich erreicht.

In einer bevorzugten Ausführungsform kann für einen Patienten sowohl eine Messreihe in Ruhe und bei körperlicher Belastung, insbesondere 150 W, aufgenommen werden, sodass die kardiovaskulären Leistungen in Abhängigkeit der entsprechenden Pulsfrequenz bestimmt werden können.

Figur 6 zeigt einen Trainingsdatensatz, exemplarisch umfassend fünf Messreihen X1 - X5 von Perfusionsamplituden, zum Trainieren des maschinellen Lernmodells 12 im Zeitverlauf. Der Trainingsdatensatz umfasst zudem vorzugsweise die Pulsfrequenz bei der die jeweilige Messreihe X1 - X5 ermittelt wurde. Der Trainingsdatensatz kann zudem eine Metrik zur Messung der körperlichen Belastung umfassen, bei der die jeweilige Messreihe aufgenommen wurde, um die Bestimmung der kardiovaskulären Leistung, die von der Pulsfrequenz abhängt, zu verbessern. Die Perfusionsamplituden der Messreihen in Fig. 6 zeigen exemplarisch sehr unterschiedliche Verläufe, da sie bei einem unterschiedlichen Grad an körperlicher Belastung und für verschiedene Patienten aufgenommen wurden. Die Messreihen X1 und X2 wurden in Ruhe aufgenommen, also ohne körperliche Belastung, während X3 - X5 während körperlicher Belastung aufgenommen wurden. Damit das maschinelles Lernmodell hinreichend gute Vorhersagen der kardiovaskulären Leistung liefert, müssen viele solcher Messreihen vorgenommen, die Zielwerte bestimmt werden, und das maschinelle Lernmodell damit trainiert werden.

Diesen Messreihen X1 - X5 sind Zielwerte der kardiovaskulären Leistung zugeordnet, die vorzugsweise durch andere bekannte Verfahren zur Bestimmung der kardiovaskulären Leistung bestimmt wurden. Die Zielwerte der kardiovaskulären Leistung können etwa durch bekannte Verfahren wie Thermodilution, das Fick-Prinzip, Doppler-Ultraschall, Pulse-Contour-Analyse, oder VO₂max Verfahren bestimmt werden. Somit kann das maschinelle Lernmodell 12 anhand dieser Messreihen X1 - X5 und Zielwerte trainiert werden, um zuverlässig die kardiovaskuläre Leistung zu bestimmen.

Der Trainingsdatensatz weist zudem Herzschlagvolumenvariabilitätswerte als Messdaten auf, sodass Schwankungen des Herzschlagvolumens, etwa durch die Atmung des Patienten, vom maschinellen Lernmodell bei der Bestimmung der kardiovaskulären Leistung berücksichtigt werden kann.

Um «overfitting» zu vermeiden, kann das maschinelle Lernmodell 12 zudem zum Validieren auf einem Validierungsdatensatz, der ebenfalls eine Vielzahl solcher Validierungsmessreihen und Validierungszielwerte umfasst, aber nicht zum Trainieren verwendet wurde, validiert werden.

## Patentansprüche

1. Ein computerimplementiertes Verfahren zur Bestimmung einer kardiovaskulären Leistung (2) umfassend die folgenden Schritte:
- Optionales Senden eines ersten Beaufschlagungsbefehls, vorzugsweise durch eine Kontrolleinheit (3), zur Beaufschlagung einer Manschette über einen systolischen Blutdruck eines Patienten, um einen Blutfluss zu einer distalen Peripherie (4) des Patienten zu unterbinden,
- Optionales Senden eines zweiten Beaufschlagungsbefehls, vorzugsweise durch die Kontrolleinheit (3), zur Beaufschlagung der Manschette (9) zu einem subsystolischen Blutdruck, um einen Blutfluss zu der distalen Peripherie (4) zu ermöglichen,
- Empfangen von einer Vielzahl von Messdaten (5) durch eine Kommunikationsschnittstelle (6) von einem Sensor (7) an einem Messabschnitt (41) der distalen Peripherie (4) des Patienten, wobei die Messdaten (5) Aufschluss über eine Perfusion des Blutflusses am Messabschnitt (41) geben, und wobei die Vielzahl der Messdaten (5) zumindest zwei verschiedene Messdaten (5) umfassen, die zu unterschiedlichen Zeiten nach dem Wiederherstellen des Blutflusses zu der distalen Peripherie (4) detektiert wurden,
- Bestimmung der kardiovaskulären Leistung (2), insbesondere des Herzzeitvolumens, anhand der Vielzahl von Messdaten,
- Bereitstellung von Ausgangsdaten durch eine Ausgangsschnittstelle (8) umfassend die kardiovaskuläre Leistung (2) .

2. Das computerimplementierte Verfahren nach Anspruch 1, wobei die Vielzahl von Messdaten (5) zur Bestimmung der kardiovaskulären Leistung (2) über ein Zeitintervall:
- aus einem Bereich von 1 s bis 60 s, insbesondere aus einem Bereich von 1 s bis 35 s, vorzugsweise aus einem Bereich von 1 s bis 25 s, ausgewählt werden, oder
- so gewählt werden, dass eine Abweichung von zeitlich direkt aufeinander folgenden Messdaten immer grösser ist als 5%, insbesondere 4%, vorzugsweise 3%,
- so gewählt werden, dass ein Mittelwert von 3, 4, oder 5 zeitlich unmittelbar aufeinander folgenden gemessenen Messdaten grösser ist als ein vordefinierter Anteil, insbesondere 75%, vorzugsweise 85%, eines zeitlich unmittelbar darauffolgend gemessenen Messwertes.

3. Das computerimplementierte Verfahren nach einem der vorhergehenden Ansprüche, wobei die Vielzahl der Messdaten folgendes umfassen oder daraus bestehen:
- Messdaten, die auf die Sauerstoffsättigung im Blut schliessen lassen, insbesondere SpO₂-Messdaten,
- Volumetrische Messdaten, die auf eine Perfusionsamplitude am Messabschnitt (41) schliessen lassen, insbesondere Perfusionsindex-Messdaten, und/oder
- plethysmographische Messdaten, die auf das Erkennen einer Pulsation schliessen lassen,
- und optional zusätzlich
- Pulsfrequenzen.

4. Das computerimplementierte Verfahren nach Anspruch 3, wobei eine Perfusionsamplitude und, insbesondere eine Herzschlagvolumenvariabilität, aus der Vielzahl von Messdaten bestimmt wird, insbesondere aus den SpO₂-Messdaten, den Perfusionsindex-Messdaten, oder den plethysmographischen Messdaten.

5. Das computerimplementierte Verfahren nach Anspruch 4, wobei das Verfahren umfasst, dass die Herzschlagvolumenvariabilität verwendet wird, um die Messdaten, insbesondere die Perfusionsamplitude, zu korrigieren.

6. Das computerimplementierte Verfahren nach einem der Ansprüche 4 oder 5, umfassend eine Bestimmung der kardiovaskulären Leistung (2) mit Hilfe mindestens eines der folgenden Algorithmen:
- zumindest einer Fit-Funktion der Perfusionsamplitude, insbesondere umfassend eine polynomische Fit-Funktion, eine logarithmische Fit-Funktion, eine exponentielle Fit-Funktion, insbesondere mit schwingender Dämpfung, eine logistische Wachstumsmodellfit-Funktion, insbesondere mit schwingender Dämpfung, eine Potenzgesetzsättigungsfit-Funktion, eine Hill Fit-Funktion, eine Differentialgleichungsfit-Funktion, insbesondere zweiter Ordnung ist,
- Bereitstellung einer Vielzahl von Referenz-Funktion, die vorzugsweise zeitlich vorher gemessenen Messdaten umfassen, insbesondere zeitlich vorher gemessene Messdaten einer ähnlichen oder der gleichen Pulsfrequenz, Bestimmung einer Abweichung der Vielzahl von Referenz-Funktionen mit der Vielzahl von empfangenen Messdaten oder von Korrelationswerten der Messdaten, und Auswahl einer Referenz-Funktion der Vielzahl von Referenz-Funktionen mit der geringsten Abweichung oder des ähnlichsten Korrelationswerts zu den empfangenen Messdaten,
- einer über ein Zeitintervall der Messung gemittelten Perfusionsamplitude, welche mit der Pulsfrequenz verrechnet wird, insbesondere vollständig oder anteilig mit der Pulsfrequenz multipliziert wird, und
- eines maschinellen Lernmodells, insbesondere eines vortrainiertes maschinellen Lernmodells, anhand der Perfusionsamplituden.

7. Das computerimplementierte Verfahren nach einem der vorhergehenden Ansprüche, wobei der zeitliche Abstand zwischen dem ersten Beaufschlagungsbefehl und dem zweiten Beaufschlagungsbefehl mindestens 15 s, insbesondere mindestens 20 s, vorzugsweise mindestens 25 s, beträgt, oder
der zeitliche Abstand so gewählt ist, sodass ein Messwert oder ein Mittelwert von 2, 3, 4, oder 5 zeitlich unmittelbar aufeinander folgenden gemessenen Messdaten, insbesondere Perfusionsindex-Messdaten, kleiner ist als ein vordefinierter Anteil, insbesondere 20 %, vorzugsweise 15 %, eines gemessenen Messwertes oder Mittelwerts von 2, 3, 4, oder 5 zeitlich unmittelbar aufeinanderfolgenden Messwerten, der vor der Druckbeaufschlagung gemessen wurden.

8. Das computerimplementierte Verfahren nach einem der vorhergehenden Ansprüche, wobei die Vielzahl von Messdaten des Sensors (7) in Ruhe und/oder unter körperlicher Belastung des Patienten detektiert werden.

9. Das computerimplementierte Verfahren nach Anspruch 8, umfassend eine erste Bestimmung einer ersten kardiovaskulären Leistung (2) anhand von Messdaten (5), die in Ruhe aufgenommen wurden und eine zweite Bestimmung einer zweiten kardiovaskulären Leistung (2) anhand von Messdaten (5), die unter körperlicher Belastung des Patienten aufgenommen wurden.

10. Computerprogrammprodukt mit Anweisungen, die, wenn das Programm von einem Computer ausgeführt wird, den Computer veranlassen, das computerimplementierte Verfahren nach einem der vorhergehenden Ansprüche auszuführen.

11. Computerlesbares Medium, das Anweisungen enthält, die, wenn sie von einem Computer ausgeführt werden, den Computer veranlassen, das computerimplementierte Verfahren nach einem der Ansprüche 1 - 9 auszuführen.

12. System (101) konfiguriert zum Durchführen des computerimplementierten Verfahrens einem der Ansprüche 1 - 9, umfassend Mittel, insbesondere eine Kommunikationsschnittstelle (6), eine Ausgangsschnittstelle (8) und eine Kontrolleinheit (3), zum Durchführen des computerimplementierten Verfahrens.

13. System (101) nach Anspruch 12, wobei das System zumindest einen Sensor (7), der mit der Kontrolleinheit (3) verbunden oder verbindbar ist, für die distale Peripherie (4) eines Patienten umfasst und, wobei der zumindest eine Sensor (7)zum Messen einer Vielzahl von Messdaten, die auf die Sauerstoffsättigung im Blut schliessen lassen, konfiguriert ist, insbesondere einen SpO₂-Sensor (7) umfasst oder daraus besteht, und/oder
der zumindest eine Sensor (7) zum Messen einer Vielzahl von volumetrischen Messdaten konfiguriert ist, insbesondere einen plethysmographischen Sensor umfasst oder daraus besteht.

14. System (101) nach einem der Ansprüche 12 - 13, wobei der zumindest eine Sensor (7):
- ein Fingerpulsoximeter,
- ein Handgelenkspulsoximeter,
- eine tragbare Uhr, insbesondere eine Smartwatch, oder ein Fitnessarmband mit integriertem Pulsoximetersensor,
- ein stationäres Pulsoximetergerät mit abnehmbaren Sensoren, und/oder
- ein Hautpflasterpulsoximeter
umfasst oder daraus besteht.

15. System (101) nach einem der Ansprüche 12 - 14, wobei das System eine Manschette (9) umfasst und die Manschette (9) durch einen Beaufschlagungsbefehl der Kontrolleinheit (3) mit Druck beaufschlagbar ist, um den Blutfluss zu der distalen Peripherie (4) des Patienten zu unterbinden und wiederherzustellen.

16. Verfahren zur Bestimmung einer kardiovaskulären Leistung (2) aus einer Vielzahl von Messdaten (5) umfassend die Schritte:
- Beaufschlagung einer Manschette (9) über einen systolischen Blutdruck eines Patienten, um einen Blutfluss zu einer distalen Peripherie (4) des Patienten zu unterbinden,
- Beaufschlagung der Manschette (9) zu einem subsystolischen Blutdruck, um einen Blutfluss zu der distalen Peripherie (4) zu ermöglichen,
- Messen einer Vielzahl von Messdaten (5) von einem Sensor (7) an einem Messabschnitt (41) der distalen Peripherie (4) des Patienten, wobei die Messdaten (5) Aufschluss über eine Perfusion am Messabschnitt (41) geben, wobei die Vielzahl der Messdaten (5) zumindest zwei verschiedene Messdaten (5) umfassen, die zu unterschiedlichen Zeiten nach dem Wiederherstellen des Blutflusses zu der distalen Peripherie (4) detektiert wurden,
- Bestimmung der kardiovaskulären Leistung (2), insbesondere des Herzzeitvolumens, anhand der Vielzahl von Messdaten (5).

17. Computerimplementiertes Verfahren zum Trainieren eines maschinellen Lernmodells (12) für die Bestimmung einer kardiovaskulären Leistung (2), insbesondere des maschinellen Lernmodells (12) nach Anspruch 6, umfassend:
- Empfangen eines Eingabetrainingsdatensatzes, der Messdaten und einen zugehörigen Zielwert einer kardiovaskulären Leistung (2) umfasst, wobei die Messdaten von zumindest einem Sensor (7) an einem Messabschnitt (41) einer distalen Peripherie (4) eines Patienten nach dem Wiederherstellen eines Blutflusses der distalen Peripherie (4) detektiert wurden, und vorzugsweise Messdaten nach Anspruch 3 sind, und
- Durchführen eines Trainingsprozesses des maschinellen Lernmodells (12) durch Vorhersagen eines Ausgabewerts, Vergleichen des Ausgabewerts mit dem Zielwert, Anpassen der Modellparameter durch eine Verlustfunktion,
- Iteratives Wiederholen des Trainingsprozesses für weitere Eingabetrainingsdatensätze bis zu einem Abbruchkriterium,
- Vorzugsweise Validieren des trainierten Modells durch Anwendung auf einen separaten Validierungsdatensatz umfassend Validierungsmessdaten und korrespondierende kardiovaskuläre Validierungszielwerte zum Überprüfen des Modells,
- Bereitstellen des trainierten Modells.

18. Trainingsdatensatz zur Verwendung in dem Verfahren zum Trainieren eines maschinellen Lernmodells (12) nach Anspruch 17, umfassend zumindest einen Eingabetrainingsdatensatz.
